# EUROPEAN PATENT APPLICATION

(11) **EP 4 434 521 A1**
(43) Date of publication of application: **25.09.2024**
(21) Application number: 22895151.3
(22) Date of filing: 04.07.2022
(51) Int. Cl.: A61K 31/4353, A61K 36/59, A61P 31/14

(54) **STEPHANIA CEPHALANTHA-DERIVED ALKALOID-CONTAINING PREPARATION**

(30) Priority: 17.11.2021 JP 2021186784
(71) Applicant: Japan as Represented by Director-General of National Institute of Infectious Diseases, Tokyo 162-8640 (JP); Sawai Pharmaceutical Co., Ltd., Yodogawa-ku Osaka-shi Osaka 532-0003 (JP)
(72) Inventor: WATASHI, Koichi, Tokyo 162-8640 (JP); SASO, Wakana, Tokyo 162-8640 (JP); TERASHIMA, Toru, Osaka City, Osaka 532-0003 (JP)
(74) Representative: dompatent von Kreisler Selting Werner - Partnerschaft von Patent- und Rechtsanwälten mbB
(86) International application number: PCT/JP2022/026543
(87) International publication number: WO 2023/089862

(57) **Abstract**

In an embodiment, a Stephania cepharantha-derived alkaloid-containing preparation for treatment of a novel coronavirus infection is provided. According to an embodiment of the present invention, a preparation for treatment of a novel coronavirus infection comprises Cepharanthine, Isotetrandrine, Cycleanine, and Berbamine. A preparation for treatment of a novel coronavirus infection is for treatment of an infection caused by the novel coronavirus mutant strain. According to an embodiment of the present invention, a Stephania cepharantha-derived alkaloid-containing preparation for treatment of a novel coronavirus infection is provided.

## Description

### TECHNICAL FIELD

An embodiment of the present invention relates to a Stephania cepharantha-derived alkaloid-containing preparation, and in particular, an embodiment of the present invention relates to a Stephania cepharantha-derived alkaloid-containing preparation for treatment of a coronavirus infection. More particularly, an embodiment of the present invention relates to a Stephania cepharantha-extracted alkaloid-containing preparation for the treatment of a novel coronavirus infection (COVID-19), comprising Cepharanthine, Isotetrandrine, Cycleanine, and Berbamine.

### BACKGROUND ART

A novel coronavirus infection (COVID-19) was identified in Wuhan City, Hubei Province, People's Republic of China in December 2019, and is an emerging infectious disease that was announced by the World Health Organization (WHO) on March 11, 2020 to be in a pandemic (global outbreak). A vaccine has been developed as a method for preventing a novel coronavirus infection, and vaccination is progressing worldwide at the time of filing of this application. At the time of filing the present application, the main therapeutic approach of the novel coronavirus infection is only symptomatic therapy, and development of a therapeutic drug is proceeding. However, no therapeutic drug has been reported that has been confirmed to be highly effective against the novel coronavirus infection.

As with the novel coronavirus infection, severe acute respiratory syndrome (SARS), reported in 2003, is known as a serious infection by coronavirus. Further, Middle East Respiratory Syndrome (MERS) due to coronavirus (MERS-CoV) named Middle East Respiratory Syndrome Coronavirus (MERS coronavirus) was reported in 2012. However, vaccines effective for prevention of the severe acute respiratory syndrome and the Middle East Respiratory Syndrome, and a therapeutic drug that has been confirmed to be highly effective have not been reported.

On the other hand, for example, Non Patent Literature 1 describes that Cepharanthine, which is one of constituents of an alkaloid extracted from Stephania cepharantha, has an antiviral effect against the SARS coronavirus. In addition, Non Patent Literature 2 describes that Cepharanthine and Tetrandrine have antiviral effects on the human coronavirus OC43 strain at an early stage of infection in cells. In Non Patent Literature 3, the inventors of the present application describe that a Cepharanthine (registered trademark) preparation (a preparation containing a plurality of alkaloids derived from Stephania cepharantha including Cepharanthine, Isotetrandrine, Cycleanine, and Berbamine) has an antiviral effect against a novel coronavirus (SARS-CoV-2 (Wuhan strain)), and that Cepharanthine (registered trademark) containing a plurality of alkaloids derived from Stephania cepharantha binds to a binding site with ACE2 of the SARS-CoV-2 spike protein, thereby inhibiting binding of the SARS-CoV-2 to the ACE2. Further, Non Patent Literature 4 describes simulation results of binding patterns of Cepharanthine, which is one of the constituents of the alkaloid extracted from Stephania cepharantha and relates to a spike protein major mutant (H49Y, D614G, and T573I) of the SARS-CoV-2 detected in Mexican individuals.

Thus, Cepharanthine, which is a mixture of alkaloids derived from Stephania cepharantha, or one of the components constituting the mixture, may be a therapeutic drug for a novel coronavirus. However, the number of reported cases of mutant strains of the novel coronavirus is increasing, and no consideration has been given to whether the alkaloids derived from Stephania cepharantha and the individual components thereof (Cepharanthine, or the like) show high efficacy against a wide range of mutant strains.

### CITATION LIST

### NON PATENT LITERATURE

Non Patent Literature 1: ZHANG Chuan-hai et. al., Antiviral activity of cepharanthine against severe acute respiratory syndrome coronavirus in vitro, Chin Med J (Engl). 2005 Mar 20;118(6):493-6.
Non Patent Literature 2: Dong Eon Kim et. al., Natural BisBenzylisoquinoline Alkaloids-Tetrandrine, Fangchinoline, and Cepharanthine, Inhibit Human Coronavirus OC43 Infection of MRC-5 Human Lung Cells, Biomolecules. 2019 Nov 4;9(11):696.
Non Patent Literature 3: Hirofumi Ohashi et. al., Potential anti-COVID-19 agents, Cepharanthine and Nelfinavir, and their usage for combination treatment. iScience. 2021 Mar 26:102367.
Non Patent Literature 4: Yudibeth Sixto-Lopez et. al., Structural insights into SARS-CoV-2 spike protein and its natural mutants found in Mexican population, Scientific Reports, 25 February 2021, 11, 1-16.

### SUMMARY OF INVENTION

### TECHNICAL PROBLEM

The present invention solves the problems described above, and one of the objects is to provide a Stephania cepharantha-derived alkaloid-containing preparation for treatment of a novel coronavirus infection. Alternatively, in an embodiment, one of the objects is to provide a Stephania cepharantha-derived alkaloid-containing preparation that can be used for treatment of an infection caused by a novel coronavirus mutant strain.

### SOLUTION TO PROBLEM

According to an embodiment of the present invention, there is provided a preparation for treatment of a novel coronavirus infection, comprising Cepharanthine, Isotetrandrine, Cycleanine and Berbamine.

The preparation for the treatment of the novel coronavirus infection may be for treatment of an infection caused by a novel coronavirus mutant strain.

### ADVANTAGEOUS EFFECT OF INVENTION

According to an embodiment of the present invention, there is provided a Stephania cepharantha-derived alkaloid-containing preparation for treatment of a novel coronavirus infection. Alternatively, according to an embodiment, there is provided a Stephania cepharantha-derived alkaloid-containing preparation, which is widely available for the treatment of infections caused by a novel coronavirus mutant strain.

### BRIEF DESCRIPTION OF DRAWINGS

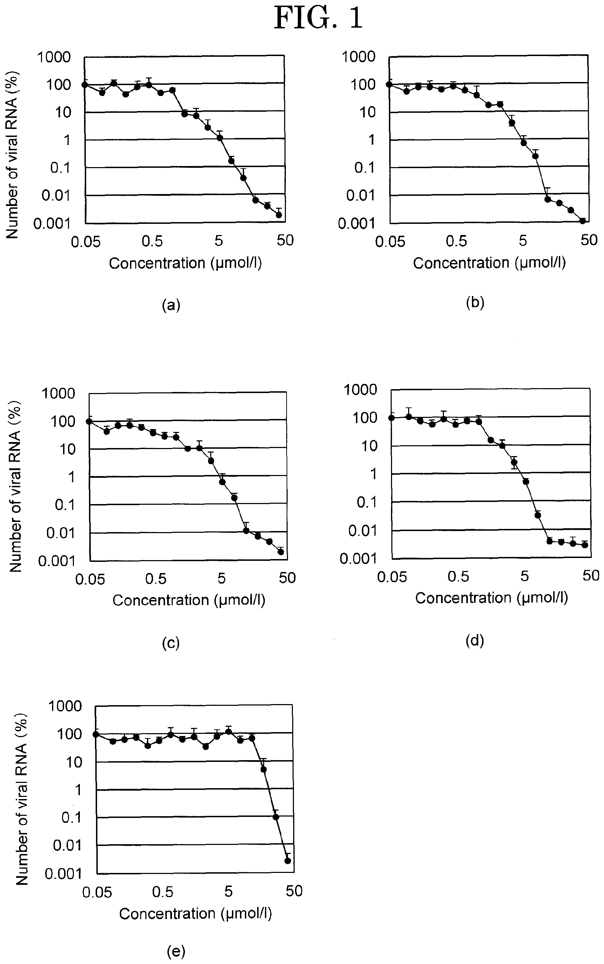

FIG. 1 shows infection test results of a novel coronavirus Wuhan strain according to an example of the present invention, (a) shows the results of a Stephania cepharantha-derived alkaloid-containing preparation, and (b) shows the results of Cepharanthine, (c) shows the results of Isotetrandrine, (d) shows the results of Cycleanine, and (e) shows the results of Berbamine.

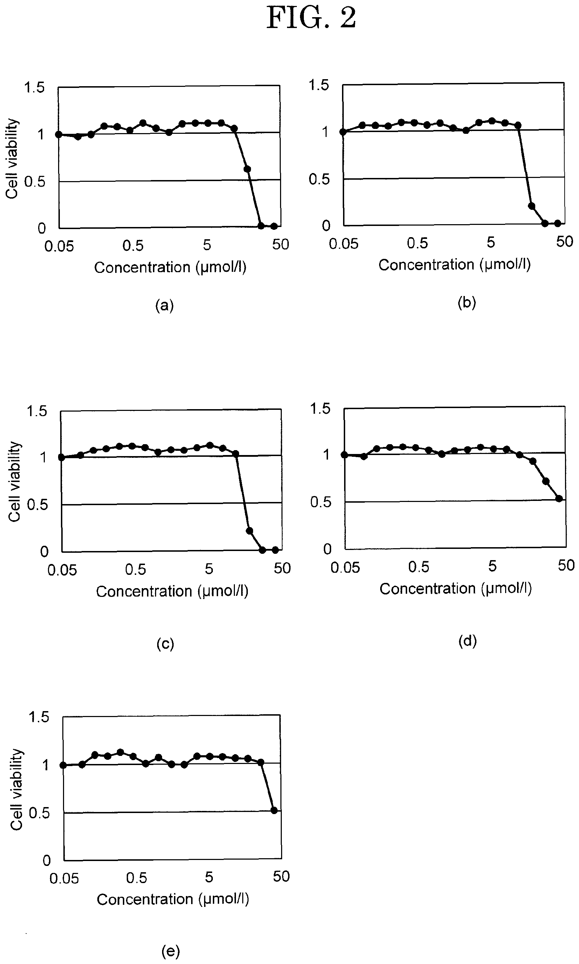

FIG. 2 shows viability of cells to which a Stephania cepharantha-derived alkaloid-containing preparation according to an example of the present invention was applied, (a) shows the results of the Stephania cepharantha-derived alkaloid-containing preparation, (b) shows the results of Cepharanthine, (c) shows the results of Isotetrandrine, (d) shows the results of Cycleanine, and (e) shows the results of Berbamine.

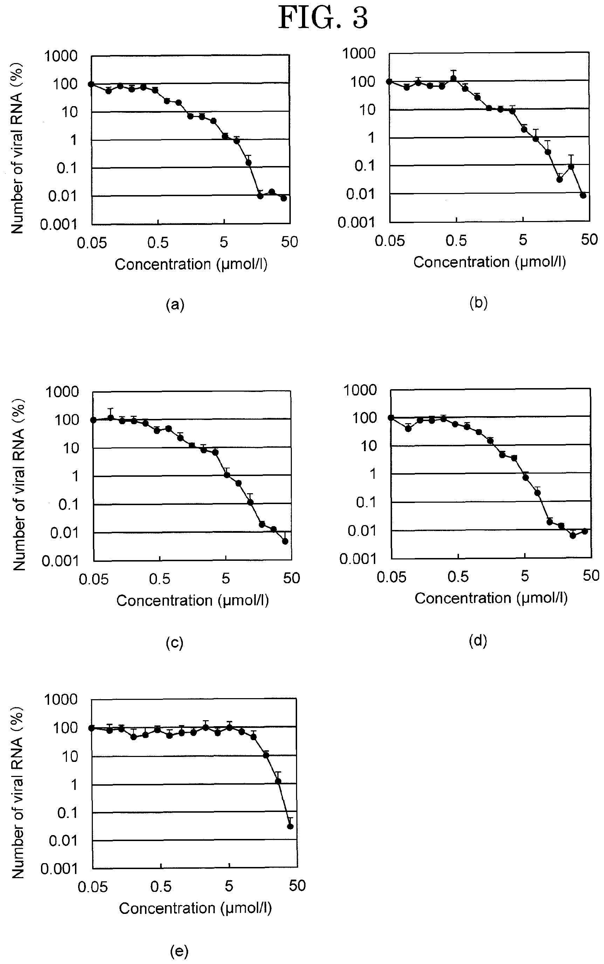

FIG. 3 shows infection test results of a novel coronavirus mutant strain (alpha mutant strain) according to an example of the present invention, (a) shows the results of a Stephania cepharantha-derived alkaloid-containing preparation, (b) shows the results of Cepharanthine, (c) shows the results of Isotetrandrine, (d) shows the results of Cycleanine, and (e) shows the results of Berbamine.

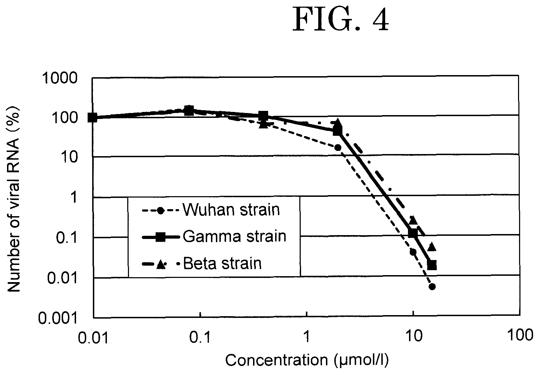

FIG. 4 shows infection test results of a novel coronavirus mutant strain (Wuhan strain, beta mutant strain, gamma mutant strain) according to an example of the present invention.

### DESCRIPTION OF EMBODIMENTS

Hereinafter, the Stephania cepharantha-derived alkaloid-containing preparation according to the present invention will be described in detail. However, the Stephania cepharantha-derived alkaloid-containing preparation of the present invention is not to be construed as being limited to the descriptions of the following embodiments and examples.

A preparation for treatment of a novel coronavirus infection according to an embodiment of the present invention includes an alkaloid derived from Stephania cepharantha. The "alkaloid derived from Stephania cepharantha" means an alkaloid extracted from the Menispermaceae family Stephania genus plant Stephania cepharantha (Stephania cepharantha Hayata). In an embodiment, a preparation for treatment of a novel coronavirus infection comprises Cepharanthine, Isotetrandrine, Cycleanine, and Berbamine, which are represented by the following chemical formulas, as a main component of an alkaloid derived from Stephania cepharantha.

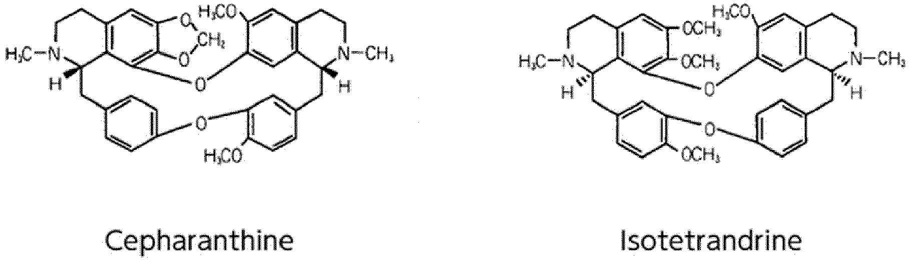

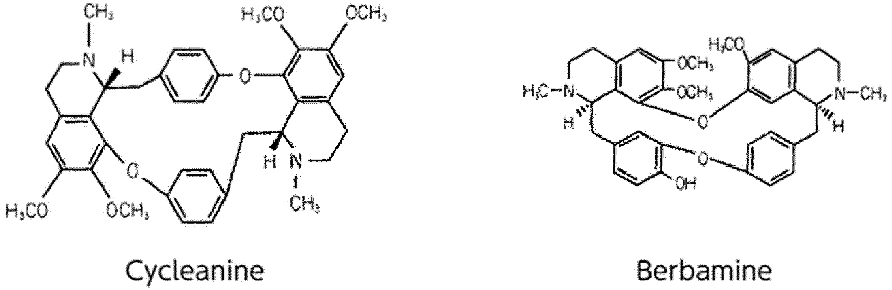

In an embodiment, a preparation for treatment of a novel coronavirus infection may comprise 19.5% to 33.5% of Cepharanthine, 25.0% to 39.0% of Isotetrandrine, 6.5% to 16.5% of Cycleanine, and 7.0% to 16.5% of Berbamine, when the total amount of alkaloids derived from the Stephania cepharantha contained in the preparation is 100% by mass. In addition, in an embodiment, a preparation for treatment of a novel coronavirus infection contains about 85% of these four alkaloids in total, when a total amount of alkaloids derived from Stephania cepharantha contained in the preparation is 100% by mass.

In an embodiment, in addition to the four alkaloids described above, a preparation for treatment of a novel coronavirus infection may comprise one or more alkaloids derived from Stephania cepharantha selected from bisbenzylisoquinoline alkaloids such as homoaromoline, cepharanoline, aromoline, obamegine, norcycleanine, 2-norcepharanthine, 2-norcepharanoline, 2-norberbamine, secocepharanthine, obaberine, 2-norisotetrandrine, oxyacanthine, stephibaberine, and thalrugosine; benzylisoquinoline alkaloids such as coclaurine, reticuline, laudanidine, protosinomenine, and N-methylcoclaurine; morphinan alkaloids such as FK-3000, sinomenine, cephamonine, tannagine, and cephamuline; aporphine alkaloids such as lastourvilline, isocorydine, and corydine; proaporphine alkaloids such as stepharine; and hasubanan alkaloids such as cepharamine, aknadinine, and aknadilactam.

Also, in an embodiment, a preparation for treatment of a novel coronavirus infection may include derivatives of these alkaloids. For example, the preparation for the treatment of the novel coronavirus infection may include an acyl derivative, an alkyl derivative, or a carbamoyl derivative in which a hydrogen atom of at least one hydroxyl group or an imino group of these alkaloids is substituted with an acyl group, an alkyl group, or a carbamoyl group.

In an embodiment, a preparation for treatment of a novel coronavirus infection may comprise 1 mg to 10 mg of the alkaloids derived from Stephania cepharantha described above per preparation. The preparation for the treatment of the novel coronavirus infection may optionally be selected from injections, powders, granules, tablets and capsules. In addition, tablets may also be uncoated tablets or film-coated tablets.

In an embodiment, a preparation for the treatment of a novel coronavirus infection may include one or more pharmaceutically acceptable additives, depending on the dosage form, in addition to the alkaloids derived from Stephania cepharantha described above. For example, the additives can be selected from excipients, disintegrants, lubricants, electrolytes, pH adjusters, preservatives, and the like, depending on the dosage form.

In an embodiment, Cepharanthin (registered trademark), Kaken Seiyaku Co., Ltd.) can be used as a preparation for treatment of a novel coronavirus infection. For example, as an injection, a Cepharanthin (registered trademark) injection 10 g sold by Kaken Seiyaku Co., Ltd., may be used. Further, for example, as a powder, Cepharanthin (registered trademark) powder 1% sold by Kaken Seiyaku Co., Ltd., may be used. Further, for example, as a tablet, a Cepharanthin (registered trademark) tablet 1 mg sold by Kaken Seiyaku Co., Ltd. may be used.

In an embodiment, for adults, 1 mg to 10 mg may be administered by intravenous injection once a day to twice a week in the case where a preparation for treatment of a novel coronavirus infection is an injection. Also, in an embodiment, for adults, 1.5 mg to 6 mg may be orally administered in two to three divided doses per day after meals in the case where the preparation for the treatment of the novel coronavirus infection is a powder or a tablet.

The preparation for the treatment of the novel coronavirus infection according to the present embodiment can be used for treatment of an infection caused by a novel coronavirus mutant strain. From the descriptions in Non Patent Documents 1 to 4, it is presumed that Cepharanthine binds to a binding site of SARS-CoV-2 spike protein with ACE2, inhibiting binding of the SARS-CoV-2 to the ACE2, thereby exerting an antiviral effect. On the other hand, mutations are likely to occur in a spike protein, and it is known that the mutations in the spike protein affect efficacy of a vaccine. The preparation for the treatment of the novel coronavirus infection according to the present embodiment, which is a mixture of a plurality of the alkaloids extracted from Stephania cepharantha, has an antiviral effect against the novel coronavirus and a mutant strain thereof.

### [Method for Manufacturing Preparation for Treatment of Novel Coronavirus Infections]

The alkaloids derived from Stephania cepharantha according to the present invention can be extracted from Stephania cepharantha by known methods. For example, it is possible to use an extract obtained by concentrating an extraction liquid from Stephania cepharantha, a precipitate produced when an acidic solution of this extract is made alkaline, an alkaloid-containing fraction separated from the precipitate, a crystal obtained by separation and purification by a known method, a derivative of an alkaloid produced by a known method, and the like. For example, Stephania cepharantha (although rhizomes, stems, seeds, leaves, and the like can be used, they are not limited to these portions) is extracted with a solvent such as methanol, ethanol, acetone, ethyl acetate, or benzene, and the extract is concentrated. The alkaloid fraction can be separated by dissolving the obtained concentrate in an acidic aqueous solution such as dilute hydrochloric acid, dilute sulfuric acid, citric acid aqueous solution, or oxalic acid aqueous solution, and then making the solution alkaline to collect the resulting precipitate. The obtained fraction may be further purified by a known means such as various chromatographic techniques or recrystallization.

Using the obtained alkaloid derived from Stephania cepharantha, any preparation selected from injections, powders, granules, tablets, or capsules can be prepared by a known manufacturing method.

### [Examples]

Hereinafter, the Stephania cepharantha-derived alkaloid-containing preparation according to the embodiment described above will be further described with reference to examples. It should be noted that the Stephania cepharantha-derived alkaloid-containing preparation according to the present invention is not limited to the following examples.

### [Example 1]

As an example 1, antiviral effects of the Stephania cepharantha-derived alkaloid-containing preparation according to the present invention and Cepharanthine, Isotetrandrine, Cycleanine, and Berbamine, which are the four main alkaloids contained in the Stephania cepharantha-derived alkaloid-containing preparation against the novel coronavirus SARS-CoV-2 (Wuhan strain) were evaluated. Cepharanthin (registered trademark)(Kaken Seiyaku Co., Ltd.) was used as a Stephania cepharantha-derived alkaloid-containing preparation. In addition, Cepharanthine, Isotetrandrine, Cycleanine, and Berbamine were also separated and purified from the Stephania cepharantha-derived alkaloid-containing preparation.

### [Cell Culture]

VeroE6/TMPRSS2 cells were cultured at 37°C in 5% of a CO₂ atmosphere by using Dulbecco's Modified Eagle's Medium (DMEM, Life Technologies Corporation) added with 10% of a fetal bovine serum (FBS, Cell Culture Bioscience Corporation), 100 units/ml of penicillin, 100 µg/ml of streptomycin, 10 mM of HEPES (pH7.4) and 1 mg/ml of G418 disulfate (Nacalai Tesque, Inc.). In infection tests, it was changed to 2% of FBS and no G418 disulfate was added.

### [Infection Tests]

Novel coronavirus SARS-CoV-2 Wk-521 strains (Wuhan strain) isolated from patients was infected with the VeroE6/TMPRSS2 cells for 1 hour at a multiplicity of infection (MOI) of 0.003. At this time, the Stephania cepharantha-derived alkaloid-containing preparation, Cepharanthine, Isotetrandrine, Cycleanine, or Berbamine was added to the medium. The cells were washed to remove viruses that did not bind to the cells. The cells were cultured for 24 hours and RNA from the novel coronavirus Wuhan strains contained in a culture supernatant was extracted using a MagMax Viral/Pathogen II Nucleic Acid Isolation kit (Thermo Fisher Scientific). The extracted RNA was quantified by a real-time RT-PCR method. Quantitative results of RNA are shown in FIG. 1(a) to FIG. 1(e).

### [Cell Viability]

The Stephania cepharantha-derived alkaloid-containing preparation, Cepharanthine, Isotetrandrine, Cycleanine, or Berbamine were added to the medium of the VeroE6/TMPRSS2 cells and, and the cells cultured for 24 hours were fixed with 4% of paraformaldehyde, and nucleuses were stained with DAPI. Thereafter, the number of viable cells were measured using an ImageXpress Micro Confocal (Molecular Device, LLC). Measurement results of cell viability are shown in FIG. 2(a) to FIG. 2(e).

FIG. 1(a) shows the results of a Stephania cepharantha-derived alkaloid-containing preparation, FIG. 1(b) shows the results of Cepharanthine, FIG. 1(c) shows the results of Isotetrandrine, FIG. 1(d) shows the results of Cycleanine, and FIG. 1(e) shows the results of Berbamine. From the results of FIG. 1(a) to FIG. 1(e), it became clear that the number of viral RNA of the novel coronavirus Wuhan strain decreased when addition concentrations of the Stephania cepharantha-derived alkaloid-containing preparation, Cepharanthine, Isotetrandrine, Cycleanine, and Berbamine were increased, and therefore, the Stephania cepharantha-derived alkaloid-containing preparation and these alkaloids showed antiviral effects against the novel coronavirus Wuhan strain.

FIG. 2(a) shows the results of the Stephania cepharantha-derived alkaloid-containing preparation, FIG. 2(b) shows the results of Cepharanthine, FIG. 2(c) shows the results of Isotetrandrine, FIG. 2(d) shows the results of Cycleanine, and FIG. 2(e) shows the results of Berbamine. From the results of FIG. 2(a) to FIG. 2(e), it became clear that the number of the viable cells decreased when the addition concentrations of the Stephania cepharantha-derived alkaloid-containing preparation, Cepharanthine, Isotetrandrine, Cycleanine, and Berbamine were increased, and therefore, the Stephania cepharantha-derived alkaloid-containing preparation and these alkaloids showed cytotoxic effects on the cells.

Comparing the results of FIG. 1(a) to FIG. 1(e) with the results of FIG. 2(a) to FIG. 2(e), it became clear that the number of viral RNA decreased at concentrations of the alkaloids with low cytotoxic effects, and therefore, the Stephania cepharantha-derived alkaloid-containing preparation, Cepharanthine, Isotetrandrine, Cycleanine, and Berbamine showed antiviral effects against the novel coronavirus Wuhan strain. Further, it became clear that Cepharanthine, Isotetrandrine, and Cycleanine showed antiviral effects against the novel coronavirus Wuhan strain comparable to the Stephania cepharantha-derived alkaloid-containing preparation. On the other hand, it became clear that Berbamine has a weak antiviral effect against the novel coronavirus Wuhan strain compared to other alkaloids.

### [Example 2]

As an example 2, antiviral effects against the novel coronavirus mutant strain (alpha mutant strain) were evaluated with respect to the Stephania cepharantha-derived alkaloid-containing preparation according to the present invention, Cepharanthine, Isotetrandrine, Cycleanine, and Berbamine in the same manner as in the example 1. Quantitative results of RNA are shown in FIG. 3(a) to FIG. 3(e).

FIG. 3(a) shows the results of the Stephania cepharantha-derived alkaloid-containing preparation, FIG. 3(b) shows the results of Cepharanthine, FIG. 3(c) shows the results of Isotetrandrine, FIG. 3(d) shows the results of Cycleanine, and FIG. 3(e) shows the results of Berbamine. From the results of FIG. 3(a) to FIG. 3(e), it became clear that the number of viral RNA of the novel coronavirus mutant (alpha mutant strain) decreased when the addition concentrations of the Stephania cepharantha-derived alkaloid-containing preparation, Cepharanthine, Isotetrandrine, Cycleanine, and Berbamine were increased, and therefore, the Stephania cepharantha-derived alkaloid-containing preparation and these alkaloids showed antiviral effects against the novel coronavirus mutant (alpha mutant strain). Further, it became clear that Cepharanthine, Isotetrandrine, Cycleanine showed antiviral effects against the novel coronavirus mutant (alpha mutant strain) comparable to the Stephania cepharantha-derived alkaloid-containing preparation. On the other hand, it became clear that Berbamine has a weak antiviral effect against the novel coronavirus mutant (alpha mutant strain) compared to other alkaloids.

### [Example 3]

As an example 3, an antiviral effect against the novel coronavirus mutant strain (gamma mutant strain) was evaluated with respect to the Stephania cepharantha-derived alkaloid-containing preparation according to the present invention by the same manner as in Example 1.

### [Example 4]

As an example 4, an antiviral effect against the novel coronavirus mutant strain (beta mutant strain) was evaluated with respect to the Stephania cepharantha-derived alkaloid-containing preparation according to the present invention by the same manner as in Example 1.

The evaluation results of the example 3 and the example 4 are shown in FIG. 4. As shown in FIG. 4, it became clear that the number of viral RNA of the novel coronavirus mutant (gamma mutant strain) and the number of viral RNA of the novel coronavirus mutant (beta mutant strain) decreased when the addition concentration of the Stephania cepharantha-derived alkaloid-containing preparation was increased, and therefore, the Stephania cepharantha-derived alkaloid-containing preparation showed antiviral effects against the novel coronavirus mutant (gamma mutant strain) and the novel coronavirus mutant (beta mutant strain).

From the results of the above examples, it became clear that the Stephania cepharantha-derived alkaloid-containing preparation according to the present invention showed antiviral effects not only against the novel coronavirus Wuhan strain but also against the novel coronavirus mutant strain (alpha mutant strain, beta mutant strain, and gamma mutant strain).

## Claims

1. A Stephania cepharantha-derived alkaloid-containing preparation for treatment of a novel coronavirus infection, comprising: Cepharanthine, Isotetrandrine, Cycleanine, and Berbamine.

2. The Stephania cepharantha-derived alkaloid-containing preparation according to claim 1, used for treatment of an infection caused by a novel coronavirus mutant strain.
